(19) **Europäisches Patentamt / European Patent Office / Office européen des brevets**

(11) **EP 4 018 921 B1**

(12) **FASCICULE DE BREVET EUROPEEN**

(45) Date de publication et mention
de la délivrance du brevet:
**12.02.2025 Bulletin 2025/07**

(21) Numéro de dépôt: **21217209.2**

(22) Date de dépôt: **22.12.2021**

(51) Classification Internationale des Brevets (IPC):
***A61B 5/022*** (2006.01)     ***A61B 5/00*** (2006.01)

(52) Classification Coopérative des Brevets (CPC):
**A61B 5/022; A61B 5/681; A61B 5/7225;
A61B 5/7239**

(54) **DISPOSITIF ET PROCÉDÉ DE CARACTÉRISATION DE LA PRESSION ARTÉRIELLE**

VORRICHTUNG UND VERFAHREN ZUR CHARAKTERISIERUNG DES BLUTDRUCKS

DEVICE AND METHOD FOR CHARACTERISING BLOOD PRESSURE.

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **27.12.2020 FR 2014163**

(43) Date de publication de la demande:
**29.06.2022 Bulletin 2022/26**

(73) Titulaire: **Commissariat à l'Energie Atomique et
aux Energies
Alternatives
75015 Paris (FR)**

(72) Inventeurs:
• **LUBIN, Mathilde
38054 Grenoble cedex 09 (FR)**
• **BONNET, Stéphane
38054 Grenoble cedex 09 (FR)**

• **GERBELOT BARILLON, Rémi
38054 Grenoble cedex 09 (FR)**

(74) Mandataire: **INNOV-GROUP
209 Avenue Berthelot
69007 Lyon (FR)**

(56) Documents cités:
**US-A1- 2010 160 798**

• **LUBIN M ET AL: "Blood pressure measurement
by coupling an external pressure and photo-
plethysmographic signals", 2020 42ND ANNUAL
INTERNATIONAL CONFERENCE OF THE IEEE
ENGINEERING IN MEDICINE & BIOLOGY
SOCIETY (EMBC), IEEE, 20 July 2020
(2020-07-20), pages 4996 - 4999, XP033816701,
DOI: 10.1109/EMBC44109.2020.9176730**

## Description

## DOMAINE TECHNIQUE

Le domaine technique est la caractérisation de la pression artérielle par une méthode optique.

## ART ANTERIEUR

La plupart des dispositifs permettant de caractériser la pression artérielle utilisent un capteur de pression couplé à un brassard de compression disposé sur un membre, généralement un bras. La caractérisation de la pression artérielle est effectuée en mesurant la pression exercée par le brassard en un ou plusieurs instants caractéristiques. Le capteur de pression ou le capteur acoustique sont sensibles aux battements cardiaques et à leur amplitude.

Dans les tensiomètres grand public, un capteur de pression détermine la pression d'air dans le brassard. La compression du brassard est effectuée de manière à obtenir une occlusion artérielle. Lors de la déflation du brassard, des oscillations de pression apparaissent. Les oscillations augmentent jusqu'à atteindre, transitoirement, une amplitude maximale. A cet instant la pression dans le brassard est considérée égale à la pression artérielle moyenne. A partir de l'amplitude maximale détectée, les instants correspondant respectivement aux pressions artérielles systolique et diastolique sont estimés sur la base de lois empiriques. Il est usuellement considéré que :

- l'instant correspondant à la pression artérielle systolique est antérieur à l'instant de la pression artérielle moyenne, et correspond à une amplitude d'oscillation d'un certain pourcentage de l'amplitude maximale, par exemple 55% ;
- l'instant correspondant à la pression artérielle diastolique est postérieur à l'instant de la pression artérielle moyenne, et correspond à une amplitude d'oscillation d'un autre pourcentage de l'amplitude maximale, par exemple 85%.

De telles méthodes, usuelles sur des tensiomètres grand publics, peuvent être contestées, notamment du fait du recours à des lois empiriques.

Les mesures optiques sont fréquemment mises en œuvre pour déterminer des paramètres physiologiques d'un être vivant. La technique de photo-pléthysmographie (PPG) est une technique connue, permettant de détecter des variations de volume de sang dans des vaisseaux sanguins dans les tissus corporels vivants. Ainsi, la PPG est une technique répandue pour estimer une fréquence cardiaque.

Le recours à des techniques optiques présente certains avantages, par exemple un coût peu élevé et une facilité d'intégration dans des dispositifs compacts, destinés à des applications grand public.

La publication Lubin M. et al «Blood pressure measurement by coupling an external pressure and photo-plethysmographic signals », EMBC, July 2020 décrit une estimation de la pression artérielle basée sur une méthode optique. Il en est de même du document US2010/0160798. Ces documents décrivent l'exploitation d'une composante de haute fréquence d'un signal optique, de type PPG, détecté.

Les inventeurs ont développé une méthode optique alternative, permettant une caractérisation de la pression artérielle.

## EXPOSE DE L'INVENTION

Un premier objet de l'invention est un procédé de caractérisation d'une pression artérielle d'un utilisateur, comportant les étapes suivantes :

- a) mise en place d'un dispositif optique sur un membre de l'utilisateur, le dispositif optique comportant :

  - une source de lumière, agencée pour émettre une lumière vers le membre ;
  - un photodétecteur, le photodétecteur étant agencé pour détecter une intensité d'une lumière émise par la source de lumière et s'étant propagée dans le membre ;

- b) compression du membre de l'individu, la compression étant effectuée entre le cœur de l'utilisateur et le dispositif optique, de telle sorte qu'une pression est appliquée sur le membre durant une durée de compression, la durée de compression comportant :

  - une phase d'inflation, au cours de laquelle la pression appliquée augmente ;
  - une phase de déflation, au cours de laquelle la pression appliquée diminue ;

- c) durant la durée de compression, illumination du membre par la source de lumière et mesure d'une intensité détectée par le photodétecteur en différents instants, de façon à obtenir une fonction temporelle correspondant à une évolution temporelle de l'intensité durant la durée de compression ;
- d) filtrage passe bas de la fonction temporelle, de façon à extraire une composante basse fréquence de la fonction temporelle ;
- e) à partir de la composante basse fréquence, caractérisation de la pression artérielle de l'individu.

Le filtrage passe bas est effectué selon une fréquence de coupure. Avantageusement, la fréquence de coupure est strictement inférieure à une fréquence cardiaque de l'individu. La fréquence de coupure est par exemple inférieure à 1 Hz ou à 0.5 Hz.

Selon un mode de réalisation, la caractérisation comporte une estimation de la pression artérielle systolique, le procédé comportant, durant la phase d'inflation :

- détection d'un instant de stabilisation au-delà duquel la composante basse fréquence se stabilise et évolue, suite à l'instant de stabilisation en formant notamment un plateau ;
- détermination de la pression appliquée à l'instant de stabilisation.

Par former un plateau, on entend que la composante basse fréquence est considérée comme stable.

Le procédé peut comporter :

- calcul d'une dérivée de la composante basse fréquence ;
- de telle sorte que l'instant de stabilisation correspond à l'instant auquel, durant l'inflation, la valeur absolue de la dérivée devient inférieure à un seuil de stabilisation.

Selon un mode de réalisation, la caractérisation comporte une estimation de la pression artérielle systolique, le procédé comportant, durant la phase de déflation :

- détection d'un instant de front auquel la composante basse fréquence débute une phase de décroissance marquée suite à une phase de stabilité ;
- détermination de la pression appliquée à l'instant de front.

Par instant de front auquel la composante basse fréquence débute une phase de décroissance marquée suite à une phase de stabilité, on entend un instant auquel l'évolution temporelle de composante basse fréquence suit une rupture de pente, passant d'une pente faible à une pente marquée.

Le procédé peut comporter :

- calcul d'une dérivée de la composante basse fréquence ;
- de telle sorte que l'instant de front correspond à l'instant auquel, durant la déflation, la valeur absolue de la dérivée devient supérieure à un seuil de front.

Selon un mode de réalisation, la caractérisation comporte une estimation de la pression artérielle moyenne, le procédé comportant, durant la phase de déflation :

- détection d'un instant auquel la composante basse fréquence atteint une pente maximale ;
- détermination de la pression appliquée audit instant.

Le procédé peut comporter :

- calcul d'une dérivée de la composante basse fréquence

- de telle sorte que l'instant d'estimation de la pression artérielle moyenne correspond à un instant auquel, durant la déflation, la valeur absolue de la dérivée est maximale.

Selon un mode de réalisation, le procédé comporte une estimation d'une pression artérielle diastolique à partir de la pression artérielle systolique et la pression artérielle moyenne.

Selon un mode de réalisation, la caractérisation comporte une estimation d'un instant auquel la composante basse fréquence atteint une valeur minimale, durant la déflation, cet instant correspondant à un instant de retour veineux. Le procédé peut comporter une détermination de la pression appliquée à l'instant de retour veineux.

Lors de l'étape c), l'illumination du membre peut être effectuée dans une bande spectrale d'illumination dans une bande spectrale comprise entre 500 nm et 1200 nm.

Un deuxième objet de l'invention est un dispositif optique de caractérisation d'une pression artérielle, le dispositif comportant :

- un lien, configuré pour fixer le dispositif optique sur un membre d'un utilisateur ;
- une source de lumière, configurée pour émettre une lumière se propageant vers le membre de l'utilisateur lorsque le dispositif est porté ;
- un photodétecteur, agencé pour détecter une lumière, émise par la source de lumière, et s'étend propagée dans le membre de l'utilisateur ;
- une unité de commande, programmé pour mettre en œuvre les étapes d) à e) d'un procédé selon le premier objet de l'invention à partir de la lumière détectée par le photodétecteur.

Les étapes d) à e) sont mises en œuvre lors d'une compression du membre. La compression peut être effectuée par un brassard de compression, disposé entre le dispositif optique et le cœur de l'utilisateur. Le brassard de compression peut faire partie du dispositif ou être associé au dispositif.

L'invention sera mieux comprise à la lecture de l'exposé des exemples de réalisation présentés, dans la suite de la description, en lien avec les figures listées ci-dessous.

## FIGURES

Les figures 1A et 1B schématisent un premier mode de réalisation d'un dispositif selon l'invention.
La figure 1C représente un autre mode de réalisation de dispositif selon l'invention.
La figure 2A montre une évolution temporelle d'une pression appliquée contre un membre durant une durée de compression.

La figure 2B montre une évolution temporelle d'une composante pulsatile (i-e haute fréquence) de l'intensité d'un faisceau lumineux rétrodiffusé par le membre lors de la compression.

La figure 3A montre une évolution temporelle de la pression mesurée au niveau d'un point de compression, ainsi qu'une évolution temporelle d'une composante basse fréquence de l'intensité d'un faisceau lumineux rétrodiffusé par le membre, en aval de la compression.

La figure 3B montre une évolution temporelle d'une composante pulsatile de l'intensité du faisceau lumineux rétrodiffusé par le membre, lors de l'essai décrit en lien avec la figure 3A.

La figure 4 schématise les principales étapes de mise en œuvre de l'invention.

## EXPOSE DE MODES DE REALISATION PARTICULIERS

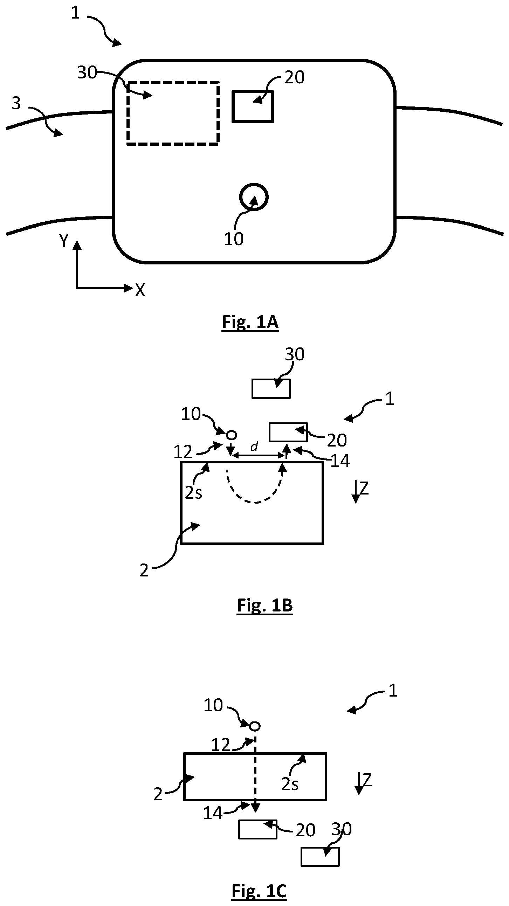

La figure 1A représente un exemple d'un dispositif optique 1 permettant une mise en œuvre de l'invention. Le dispositif optique 1 est destiné à être appliqué contre un membre 2 d'un utilisateur, humain ou animal. Le membre 2 est par exemple un bras, un avant-bras, un poignet, un doigt, une jambe. Le membre contre lequel le dispositif optique est destiné à être appliqué peut être comprimé, de façon à bloquer temporairement une circulation du sang. Dans l'exemple représenté, le dispositif 1 est relié à un lien 3, de type bracelet, de façon à être fixé contre un poignet. Il peut par exemple être intégré à une montre.

Le dispositif 1 comporte une source de lumière 10, configurée pour émettre un faisceau lumineux vers le membre 2 face auquel la source de lumière est disposée. La source de lumière 10 émet un faisceau lumineux incident 12 se propageant au membre 2, selon un axe de propagation Z. Les photons du faisceau lumineux incident 12 pénètrent dans le membre et une partie d'entre eux est rétrodiffusée, par exemple selon une direction parallèle à l'axe de propagation Z, dans un sens opposé à ce dernier. Les photons rétrodiffusés constituent un rayonnement rétrodiffusé 14. Le rayonnement rétrodiffusé 14 peut être détecté par un photodétecteur 20, placé en regard d'une surface 2s du membre. Le photodétecteur 20 peut être configuré de façon à détecter un rayonnement rétrodiffusé émanant de l'échantillon à une distance $d$, dite distance de rétrodiffusion, généralement non nulle et inférieure à quelques millimètres, typiquement inférieure à 15 mm ou 10 mm. Le photodétecteur 20 permet de mesurer l'intensité du rayonnement rétrodiffusé.

La figure 1B schématise les principaux composants du dispositif 1 dans un plan perpendiculaire à la surface 2s du membre. La flèche courbe en pointillés représente un trajet optique de photons émis par la source de lumière, dans le membre analysé.

Dans l'exemple représenté, la source de lumière émet dans une bande spectrale centrée sur 660 nm, la largeur de bande étant de 10 nm. De préférence, la bande spectrale est comprise entre 500 nm et 1200 nm, et encore de préférence entre 600 nm et 1200 nm. Dans l'exemple qui suit, la source émet dans une bande spectrale centrée sur 660 nm, la largeur de bande étant de 10 nm.

Selon une possibilité, la source de lumière peut comporter des sources de lumière élémentaires émettant dans différentes bandes spectrales.

Le dispositif optique 1 comporte une unité de traitement 30 configurée pour traiter un signal détecté par le photodétecteur 20. L'unité de traitement 30 est reliée à une mémoire, dans laquelle sont stockées des instructions pour mettre en œuvre le procédé décrit par la suite. L'unité de traitement peut comporter un microprocesseur.

Selon une alternative, représentée sur la figure 1C, le membre 2 s'étend entre la source de lumière 10 et le photodétecteur 20. Selon une telle configuration, dite en transmission, le photodétecteur 20 mesure une intensité des photons ayant traversé le membre 2. Une telle configuration suppose que le membre soit suffisamment fin pour qu'une quantité suffisante de photons émane du milieu et soit détectée par le photodétecteur.

Quel que soit le mode de réalisation, le photodétecteur 20 est agencé pour mesurer une intensité d'un faisceau de lumière formé par des photons s'étant propagés à travers le membre 2 : il s'agit soit de photons rétrodiffusés, soit de photons ayant traversé le milieu. Dans la suite de la description, on se base sur la configuration en rétrodiffusion représentée sur les figures 1A et 1B.

La mise en œuvre de l'invention suppose que le membre 2, contre lequel le dispositif 1 est appliqué, soit momentanément comprimé, de façon à réduire, voire bloquer, la circulation sanguine de la partie du membre contre laquelle le dispositif 1 est disposé. La compression est effectuée, sur le membre de l'utilisateur, en amont d'une position du dispositif sur le membre, le terme amont étant à considérer en prenant en compte la circulation artérielle. Ainsi, la compression est effectuée entre le cœur de l'utilisateur et la position du dispositif sur le membre. Dans cet exemple, le dispositif est placé au niveau du poignet d'une personne, dont le bras peut être comprimé par un brassard de compression. Il peut par exemple s'agir d'un brassard utilisé dans les tensiomètres tels que décrits dans l'art antérieur. Le brassard peut être intégré au dispositif. L'unité de traitement 30 est de préférence reliée au brassard 3, de telle sorte que l'unité de traitement reçoit une information selon laquelle le brassard est en phase d'inflation ou de déflation.

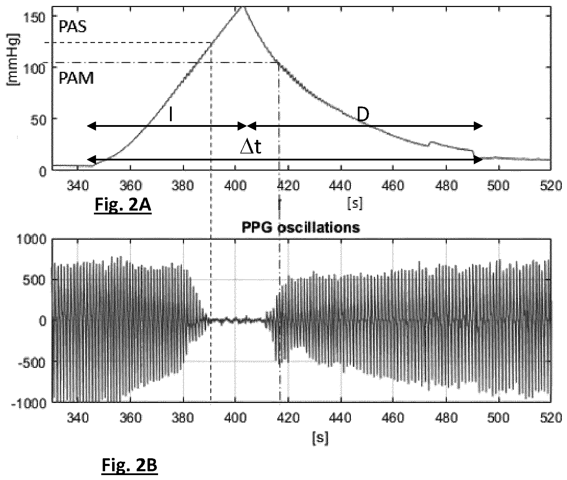

La figure 2A montre une variation d'une pression $P(t)$ appliquée contre le membre 2 à l'aide du brassard de compression. L'axe des abscisses correspond au temps. L'axe des ordonnées représente la pression appliquée. Le brassard permet une compression du membre durant une durée de compression $\Delta t$, cette dernière pouvant durer quelques dizaines de secondes, par exemple de l'ordre de 1 minute. De façon classique dans la mesure d'une tension artérielle, la compression comporte une phase d'inflation I, au cours de laquelle la pression appliquée augmente, suivie d'une phase de déflation D, au cours de laquelle la pression appliquée diminue.

Au cours de la déflation, et de préférence également au cours de l'inflation, le membre est illuminé par la source de lumière 10, et on mesure l'intensité du faisceau rétrodiffusé 14, c'est-à-dire une intensité de photons émis par la source de lumière et s'étant propagés à travers le membre examiné. L'intensité du faisceau rétrodiffusé $I(t)$ comporte une composante continue $I_{DC}(t)$, ou basse fréquence, ainsi qu'une composante pulsatile $I_{AC}(t)$, ou composante haute fréquence, due à une variation périodique du volume des vaisseaux sous l'effet des battements cardiaques. La figure 2B montre la composante pulsatile $I_{AC}(t)$ de l'intensité $I(t)$. La figure 2B montre également des variations de pression mesurées, au niveau du brassard, par un capteur de pression. Sous l'effet des battements cardiaques, les vaisseaux sanguins disposés face à la source de lumière sont soumis à une variation périodique de leur volume. Or, l'intensité détectée $I(t)$ par le photodétecteur est une image de la variation de quantité de sang dans les tissus faisant face au dispositif optique 1. Plus le volume sanguin est important, plus l'absorption est importante et plus l'intensité détectée est faible. De ce fait, en présence d'une circulation sanguine, l'intensité pulsatile mesurée par le photodétecteur subit des variations périodiques selon une fréquence correspondant à la fréquence cardiaque. Autrement dit, la composante pulsatile $I_{AC}(t)$ comporte des oscillations représentatives des battements cardiaques.

Lorsque la pression exercée par le brassard atteint et dépasse la pression artérielle systolique (PAS), la circulation sanguine s'interrompt. La composante pulsatile $I_{AC}(t)$ devient négligeable. Après que la pression exercé par le brassard a entraîné l'interruption de la circulation sanguine, la pression exercé atteint un niveau maximal, suprasystolique, puis décroît progressivement, jusqu'à une reprise de la circulation sanguine. Lors de la déflation, lorsque la pression exercée par le brassard devient inférieure à la pression systolique, la composante pulsatile $I_{AC}(t)$ suit à nouveau des oscillations. L'amplitude des oscillations augmente, jusqu'à atteindre un maximum, situé autour de 420s sur la figure 2B. La pression mesurée à cet instant est la pression artérielle moyenne (PAM).

De manière assez analogue aux mesures usuelles, basées sur des oscillations de la pression du brassard, la composante pulsatile permet de déterminer un instant auquel les oscillations sont maximales. A cet instant, on peut considérer que la pression du brassard correspond à la pression artérielle moyenne. Les courbes représentées sur les figures 2A et 2B montrent que l'analyse de la composante pulsatile $I_{AC}(t)$ permet d'estimer l'instant auquel la pression exercée sur le membre correspond à la pression artérielle moyenne (PAM).

Le recours à une composante pulsatile d'un signal optique rétrodiffusé, pour estimer la pression artérielle, a été décrit dans la publication Lubin M. et al «Blood pressure measurement by coupling an external pressure and photo-plethysmographic signals », EMBC, July 2020. Cependant, les inventeurs considèrent qu'un inconvénient de cette méthode est que la composante pulsatile $I_{AC}(t)$ peut être « noyée » dans des variations de la composante continue $I_{DC}(t)$. Ainsi, la composante pulsatile extraite peut être affectée d'un mauvais rapport signal sur bruit. De plus, les inventeurs ont constaté que les mesures de la composante pulsatile sont sensibles au positionnement du dispositif sur l'utilisateur.

Les inventeurs ont considéré qu'il était préférable d'analyser non pas la composante pulsatile $I_{AC}(t)$ de l'intensité détectée par le capteur 20, mais la composante continue, c'est-à-dire une composante basse fréquence $I_{DC}(t)$. Cette composante n'est pas utilisée pour estimer la pression artérielle. En effet, la composante pulsatile $I_{AC}(t)$ est considérée comme plus informative, du fait des similitudes avec les oscillations de pression mesurées lors de la déflation d'un brassard.

Aussi, et il s'agit d'un élément important de l'invention, l'intensité « basse fréquence » $I_{DC}(t)$ du faisceau 14, « débarrassée » de la composante pulsatile, s'avère être un indicateur pertinent, permettant une estimation d'instants auxquels la pression exercée par le brassard correspond à la pression artérielle systolique ou à la pression artérielle moyenne ou à d'autres pressions artérielles caractéristiques.

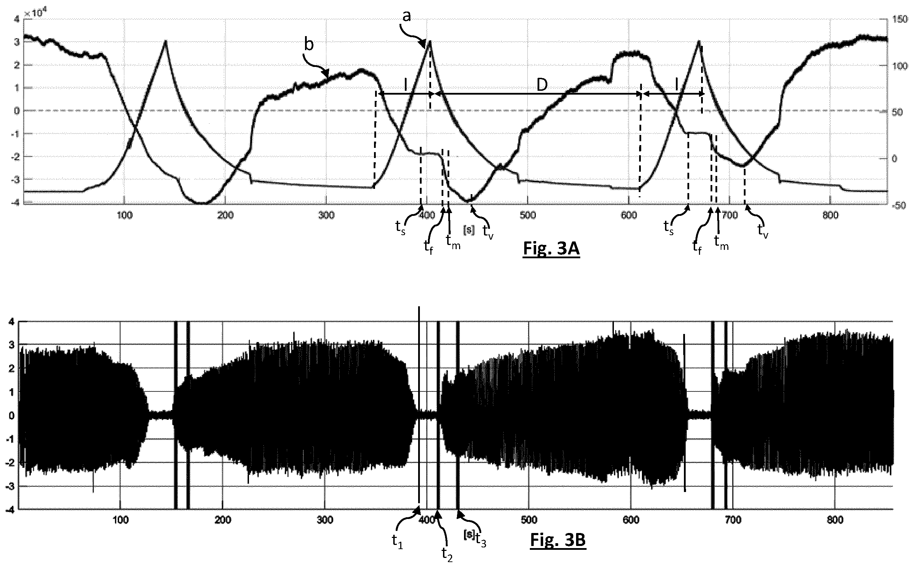

La figure 3A représente la pression exercé par le brassard sur le bras de l'utilisateur (courbe a - axe des ordonnées à droite - unité arbitraire) en fonction du temps (axe des abscisses - unité seconde). On a représenté, sur la figure 3A, plusieurs cycles d'inflation I / déflation D. Au cours de chaque inflation et de chaque déflation, le bras est illuminé par la source de lumière 10 et le photodétecteur 20 mesure une intensité d'une lumière rétrodiffusée. L'intensité $I(t)$ de la lumière rétrodiffusée est mesurée selon une fréquence d'échantillonnage de préférence supérieure à 10 Hz, voire supérieure à 100 Hz. Plus la fréquence d'échantillonnage est élevée, meilleure est la résolution temporelle des mesures. On obtient ainsi une variation temporelle $I(t)$ de l'intensité durant la durée de compression $\Delta t$. La variation temporelle $I(t)$ mesurée fait l'objet d'un filtrage passe bas, de façon à ne conserver que des composantes fréquentielles inférieures à une fréquence de coupure. On obtient ainsi la composante basse fréquence $I_{DC}(t)$. Afin d'éliminer la composante périodique décrite en lien avec la figure 2B, la fréquence de coupure est strictement inférieure à la fréquence cardiaque de l'utilisateur. La fréquence de coupure est généralement inférieure à 1 Hz. Elle peut être comprise entre 0,1 Hz et 1 Hz. Elle peut être par exemple de quelques 0.1 Hz, par exemple 0.5 Hz. Sur la figure 3A, on a représenté une évolution de la composante basse fréquence $I_{DC}(t)$ durant chaque cycle d'inflation/déflation : cf. courbe (b) - axe des ordonnées à gauche - unité arbitraire.

La composante basse fréquence $I_{DC}(t)$ reflète la quantité de sang présente dans les tissus situés face au dispositif optique 1. Durant l'inflation, la circulation de sang diminue peu à peu, jusqu'à être bloquée. L'accumulation de sang entraîne une diminution du signal rétrodiffusé, et donc de la composante basse fréquence $I_{DC}(t)$ du fait d'une augmentation de l'absorption des photons par le sang s'accumulant en aval de la compression. Lorsque la circulation sanguine est bloquée, la composante basse fréquence se stabilise, en formant un plateau. Ce plateau correspond à une relative stagnation de l'intensité $I_{DC}(t)$. Le plateau s'étend entre :

- un instant de stabilisation $t_s$ qui correspond à l'instant auquel la pression exercée par le brassard, lors de l'inflation, atteint puis dépasse la pression artérielle systolique PAS ;
- un instant de front $t_f$, postérieur à l'instant de stabilisation, qui correspond à l'instant auquel la pression exercée par le brassard, lors de la déflation, atteint la pression artérielle systolique et devient inférieur à cette dernière.

Entre le début de l'inflation et l'instant de stabilisation $t_s$, la dérivée $I'_{DC}(t)$ de $I_{DC}(t)$ est négative, ce qui traduit une décroissance de $I_{DC}(t)$. L'instant de stabilisation $t_s$ correspond à l'instant auquel durant l'inflation, la dérivée $I'_{DC}(t)$ s'annule, ou, de façon plus générale, la valeur absolue $|I'_{DC}(t)|$ de la dérivée $I'_{DC}(t)$ devient inférieure à un seuil, dit seuil de stabilisation $Th_s$. L'instant de stabilisation correspond à un instant auquel $I_{DC}(t)$, durant l'inflation, passe d'une décroissance marquée à une décroissance modérée, voire une stabilisation.

Ainsi, à $t_s$,

$$|I'_{DC}(t_s)| \leq Th_s \quad (1).$$

L'instant de front $t_f$ correspond à un instant auquel, durant la déflation, la dérivée $I'_{DC}(t)$ de la composante basse fréquence est négative, et que la valeur absolue de la dérivée $|I'_{DC}(t)|$ devient supérieure à un seuil de front $Th_f$. Le seuil de front peut avoir la même valeur que le seuil de stabilisation évoqué dans le paragraphe précédent. L'instant de front correspond à un instant auquel $I_{DC}(t)$, durant la déflation, passe relative stabilité à une décroissance marquée, formant un front.

Ainsi, à $t_f$

$$\left|I'_{DC}(t_f)\right| \geq Th_f \quad (2)$$

Entre l'instant de stabilisation $t_s$ et l'instant de font $t_f$, le brassard exerce une pression supérieure à la pression artérielle systolique : la pression exercée est dite supra-systolique.

Lors de la déflation, après l'instant de front $t_f$, la circulation sanguine reprend, tout d'abord par les artères. Cependant, les veines, plus souples que les artères, restent temporairement occluses par la pression exercée par le brassard. Il en résulte une accumulation supplémentaire de sang, se traduisant par une diminution importante de l'intensité du signal rétrodiffusé. Le signal détecté atteint une pente maximale, à un instant de pente maximale $t_m$. L'instant $t_m$ est considéré comme l'instant auquel la pente de $I_{DC}(t)$ est maximale au cours de la déflation. Cela correspond à une valeur maximale de $|I'_{DC}(t)|$ au cours de la déflation. A cet instant, on considère que la pression exercée par le brassard correspond à la pression artérielle moyenne PAM : l'artère est dite compliante.

Au cours de la déflation, lorsque l'occlusion veineuse cesse, ce qui est désigné par le terme « retour veineux », la quantité de sang face au dispositif diminue. L'absorption des photons par le sang diminue également, ce qui entraîne une augmentation de l'intensité détectée. Cela correspond à l'instant où, lors de la déflation, la dérivée $I'_{DC}(t)$ redevient positive. L'instant de retour veineux est noté $t_v$. Il correspond à l'instant où $I_{DC}(t)$ est minimal. On peut alors mesurer la pression du brassard à l'instant de retour veineux $t_v$.

Sur la figure 3B, on a représenté la composante pulsatile $I_{AC}(t)$ du signal détecté (axe des ordonnées - unité arbitraire) en fonction du temps (axe des abscisses - unité seconde - même référentiel temporel que la figure 3A). On observe des instants $t_1$ et $t_2$, qui correspondent respectivement à l'arrêt des oscillations lors de l'inflation et à la reprise des oscillations lors de la déflation. Ces instants correspondent respectivement aux instants $t_s$ et $t_f$ décrits en lien avec la figure 3A. Sur la figure 3B, on a également représenté un instant $t_3$, qui correspond à la pression diastolique. Les instants $t_1$, $t_2$ et $t_3$ ont été déterminés par un médecin.

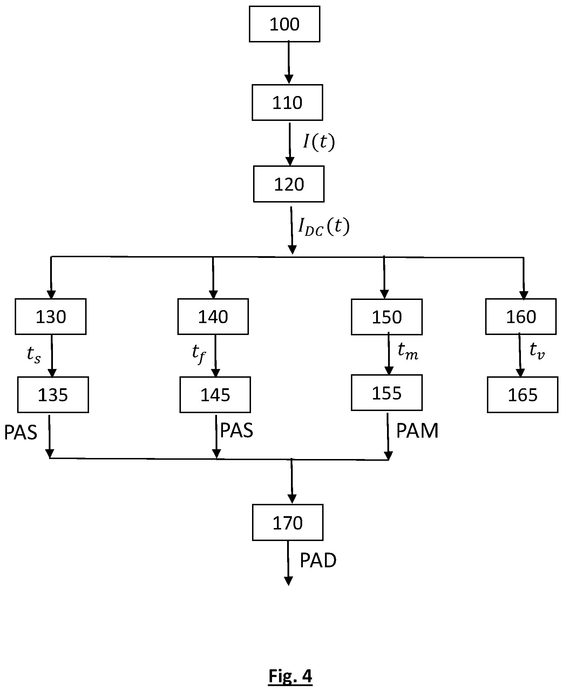

La figure 4 schématise les principales étapes d'un procédé selon l'invention, décrites ci-après :

Etape 100 : Application d'une pression au membre 2, en amont du dispositif optique, durant une durée de compression. Le terme amont s'entend selon le sens de la circulation artérielle. La durée de compression comporte une phase d'inflation I, au cours de laquelle la pression augmente, et une phase de déflation D, au cours de laquelle la pression diminue.

Etape 110 : Mesure de l'intensité de la lumière rétrodiffusée ou transmise par le membre 2 durant la compression . On obtient ainsi une fonction temporelle, représentant une variation de l'intensité $I(t)$ durant compression.

Etape 120 : Filtrage passe bas de la fonction $I(t)$, la fréquence de coupure du filtre étant strictement inférieure à la fréquence cardiaque de l'utilisateur. On obtient ainsi la composante basse fréquence $I_{DC}(t)$ de l'intensité mesurée, ou intensité basse fréquence. Le filtrage peut être obtenu par moyenne glissante, filtrage gaussien, ou par un filtre passe bas plus raffiné.

Les étapes 130, 135 ou 140, 145, décrites par la suite, visent à déterminer la pression artérielle systolique. On met en œuvre soit les étapes 130 et 135, soit les étapes 140 et 145, soit l'ensemble de ces étapes. Lorsqu'on met en œuvre l'ensemble de ces étapes, on obtient deux estimations de la pression systolique, que l'on peut moyenner, ou dont on ne retient que l'estimation considérée comme la plus fiable.

Etape 130 : Durant l'inflation, détection de l'instant de stabilisation $t_s$, au cours duquel la composante basse fréquence $I_{DC}(t)$, décroissante, atteint un plateau, ou, de façon plus générale, peut être considérée comme stable. L'instant de stabilisation peut être détecté comme correspondant à un instant auquel durant l'inflation, la valeur absolue $|I'_{DC}(t)|$ de la dérivée $I'_{DC}(t)$ devient inférieure au seuil de stabilisation $Th_s$, ce seuil pouvant par exemple être 0 ou proche de 0. Le seuil $Th_s$ peut être prédéterminé.

Etape 135 : Mesure de la pression appliquée à l'instant de stabilisation $t_s$ résultant de l'étape 130. La pression mesurée correspond à la pression artérielle systolique PAS. Autrement dit, $PAS = P(t_s)$.

Etape 140 : Durant la phase de déflation, détection de l'instant de front $t_f$, qui correspond à l'atteinte de la pression systolique. L'instant de front correspond à un instant auquel durant la déflation, la valeur absolue $|I'_{DC}(t)|$ de la dérivée $I'_{DC}(t)$ devient supérieure au seuil de front $Th_f$. Le seuil $Th_f$ peut être prédéterminé.

Etape 145 : Mesure de la pression appliquée à l'instant de front $t_f$. La pression mesurée correspond à la pression artérielle systolique PAS. Autrement dit, $PAS = P(t_f)$.

Etape 150 : Durant la phase de déflation, détection de l'instant $t_m$ auquel la pente de $I_{DC}(t)$ est maximale. Cet instant qui correspond à l'atteinte de la pression artérielle moyenne.

Etape 155 : Mesure de la pression appliquée à l'instant $t_m$. La pression mesurée correspond à la pression artérielle moyenne PAM. Autrement dit, $PAM = P(t_m)$.

Etape 160 : Durant la phase de déflation, détection de l'instant $t_v$ auquel la valeur $I_{DC}(t)$ est minimale. Cet instant qui correspond à l'instant de retour veineux.

Etape 165 : Mesure de la pression à l'instant $t_v$.

Etape 170 : Au cours de cette étape, à partir de PAM résultant de l'étape 155, et de PAS résultant de l'étape 135 et/ou de l'étape 145, on détermine la pression artérielle diastolique, selon une relation empirique :

$$PAD = \frac{3}{2} PAM - \frac{1}{2} PAS \quad (3)$$

Les étapes 130/135, 140/145, 150/155 et 160/165 peuvent être mises en œuvre indépendamment les unes des autres. Le procédé peut comporter toutes ces étapes, ou uniquement une partie de ces étapes. Par exemple, si l'on ne recherche que la PAM, seules les étapes 150 et 155 sont mises en œuvre après l'étape 120.

L'invention permet une caractérisation fiable de la tension artérielle, et cela à l'aide de composants optiques simples, pouvant être intégrés dans des dispositifs nomades, portés par l'utilisateur : il peut par exemple s'agir de montres ou de brassards instrumentés.

## Revendications

1. Procédé de caractérisation d'une pression artérielle d'un utilisateur, comportant les étapes suivantes :

   - a) disposition d'un dispositif optique (1) sur un membre (2) de l'utilisateur, le dispositif optique comportant :

     • une source de lumière (10), agencée pour émettre une lumière (12) vers le membre ;
     • un photodétecteur (20), le photodétecteur étant agencé pour détecter une intensité d'une lumière (14) émise par la source de lumière et s'étant propagée dans le membre ;

   - b) compression du membre de l'utilisateur, la compression étant effectuée entre le cœur de l'individu et le dispositif optique (1), de telle sorte qu'une pression ($P(t)$) est appliquée sur le membre durant une durée de compression ($\Delta t$), la durée de compression comportant :

     • une phase d'inflation, au cours de laquelle la pression appliquée augmente ;
     • une phase de déflation, au cours de laquelle la pression appliquée diminue ;

   - c) durant la durée de compression ($\Delta t$), illumination du membre par la source de lumière et mesure d'une intensité détectée par le photodétecteur en différents instants, de façon à obtenir une fonction temporelle ($I(t)$) correspondant à une évolution temporelle de l'intensité durant durée de compression ;

le procédé étant **caractérisé en ce qu'**il comporte également les étapes :

- d) filtrage passe bas de la fonction temporelle ($I(t)$), de façon à extraire une composante basse fréquence ($I_{DC}(t)$) de la fonction temporelle ($I(t)$), le filtrage passe-bas étant effectué selon une fréquence de coupure strictement inférieure à une fréquence cardiaque de l'individu ;
- e) à partir d'un instant déterminé sur la composante basse fréquence ($I_{DC}(t)$), caractérisation de la pression artérielle de l'individu en fonction de la pression appliquée audit instant.

2. Procédé selon la revendication 1, dans lequel la fréquence de coupure est inférieure à 1 Hz ou à 0.5 Hz.

3. Procédé selon l'une quelconque des revendications précédentes, dans lequel la caractérisation comporte une estimation de la pression artérielle systolique (PAS), le procédé comportant, durant la phase d'inflation :

- détection d'un instant de stabilisation ($t_s$) au-delà duquel la composante basse fréquence ($I_{DC}(t)$) se stabilise et évolue, suite à l'instant de stabilisation, en formant un plateau ;
- détermination de la pression ($P(t_s)$) appliquée à l'instant de stabilisation.

4. Procédé selon la revendication 3, comportant :

- calcul d'une dérivée de la composante basse fréquence ($I'_{DC}(t)$) ;
- de telle sorte que l'instant de stabilisation ($t_s$) correspond à l'instant auquel, durant l'inflation, la valeur absolue de la dérivée ($|I'_{DC}(t)|$) devient inférieure à un seuil de stabilisation ($Th_s$).

5. Procédé selon l'une quelconque des revendications précédentes, dans lequel la caractérisation comporte une estimation de la pression artérielle systolique (PAS), le procédé comportant, durant la phase de déflation :

- détection d'un instant de front ($t_f$) auquel la composante basse fréquence ($I_{DC}(t)$) débute une phase de décroissance marquée suite à une phase de stabilité ;
- détermination de la pression ($P(t_f)$) appliquée à l'instant de front.

6. Procédé selon la revendication 5, comportant :

- calcul d'une dérivée de la composante basse fréquence ($I'_{DC}(t)$) ;
- de telle sorte que l'instant de front ($t_f$) correspond à l'instant auquel, durant la déflation, la valeur absolue de la dérivée ($|I'_{DC}(t)|$) devient supérieure à un seuil de front ($Th_f$).

7. Procédé selon l'une quelconque des revendications précédentes dans lequel la caractérisation comporte une estimation de la pression artérielle moyenne (PAM), le procédé comportant, durant la phase de déflation :

- détection d'un instant $^{™}$ auquel la composante basse fréquence ($I_{DC}(t)$) atteint une pente maximale;
- détermination de la pression ($P(t_m)$) appliquée audit instant $^{™}$.

8. Procédé selon la revendication 5, comportant :

- calcul d'une dérivée de la composante basse fréquence ($I'_{DC}(t)$) ;
- de telle sorte que l'instant d'estimation de la pression artérielle moyenne ($t_m$) correspond à un instant auquel, durant la déflation, la valeur absolue de la dérivée ($|I'_{DC}(t)|$) est maximale.

9. Procédé selon l'une quelconque des revendications 3 à 6 et selon la revendication 7, comportant une estimation d'une pression artérielle diastolique (PAD) à partir de la pression artérielle systolique (PAS) et la pression artérielle moyenne (PAM).

10. Procédé selon l'une quelconque des revendications précédentes dans lequel la caractérisation comporte une estimation d'un instant ($t_v$) auquel la composante basse fréquence ($I_{DC}(t)$) atteint une valeur minimale, durant la déflation, cet instant correspondant à un instant de retour veineux.

11. Procédé selon la revendication 10, comportant une détermination de la pression ($P(t_v)$) appliquée à l'instant de retour veineux.

12. Procédé selon l'une quelconque des revendications précédentes, dans lequel lors de l'étape c), l'illumination du membre est effectuée dans une bande spectrale d'illumination dans une bande spectrale comprise entre 500 nm et 1200 nm.

13. Dispositif optique (1) de caractérisation d'une pression artérielle, le dispositif comportant :

- un lien (3), configuré pour fixer le dispositif optique sur un membre (2) d'un utilisateur ;

- une source de lumière (10), configurée pour émettre une lumière (12) se propageant vers le membre de l'utilisateur lorsque le dispositif est porté ;
- un photodétecteur (20), agencé pour détecter une lumière (14), émise par la source de lumière, et s'étend propagée dans le membre de l'utilisateur ;
- une unité de commande (30), programmé pour mettre en œuvre les étapes d) à e) selon l'une quelconque des revendications précédentes à partir de la lumière détectée par le photodétecteur.

**Patentansprüche**

1. Verfahren zur Charakterisierung eines Blutdrucks eines Benutzers, umfassend die folgenden Schritte:

   - a) Anordnen einer optischen Vorrichtung (1) an einer Gliedmaße (2) des Benutzers, wobei die optische Vorrichtung umfasst:

      • eine Lichtquelle (10), die dazu gestaltet ist, ein Licht (12) zu der Gliedmaße hin zu emittieren,
      • einen Fotodetektor (20), wobei der Fotodetektor dazu gestaltet ist, eine Intensität eines Lichts (14) zu messen, das von der Lichtquelle emittiert wird und sich in der Gliedmaße ausgebreitet hat;

   - b) Kompression der Gliedmaße des Benutzers, wobei die Kompression zwischen dem Herz der Person und der optischen Vorrichtung (1) so durchgeführt wird, dass ein Druck ($P(t)$) auf die Gliedmaße während einer Kompressionsdauer ($\Delta t$) ausgeübt wird, wobei die Kompressionsdauer umfasst:

      • eine Inflationsphase, in deren Verlauf der ausgeübte Druck steigt;
      • eine Deflationsphase, in deren Verlauf der ausgeübte Druck sinkt;

   - c) während der Kompressionsdauer ($\Delta t$), Beleuchten der Gliedmaße durch die Lichtquelle und Messen einer Intensität, die von dem Fotodetektor zu verschiedenen Zeitpunkten detektiert wird, so dass eine Zeitfunktion ($I(t)$) erhalten wird, die einem zeitlichen Verlauf der Intensität während der Kompressionsdauer entspricht, wobei das verfahren **dadurch gekennzeichnet ist, dass** es umfasst :
   - d) Tiefpassfilterung der Zeitfunktion ($I(t)$), so dass eine Niederfrequenzkomponente ($I_{DC}(t)$) der Zeitfunktion ($I(t)$) extrahiert wird, wobei die Tiefpassfilterung gemäß einer Grenzfrequenz durchgeführt wird, die strikt kleiner als eine Herzfrequenz der Person ist;
   - e) ab einem an der Niederfrequenzkomponente ($I_{DC}(t)$) bestimmten Zeitpunkt, Charakterisierung des Blutdrucks der Person in Abhängigkeit von dem zu diesem Zeitpunkt ausgeübten Druck.

2. Verfahren nach 1, bei dem die Grenzfrequenz kleiner als 1 Hz oder als 0,5 Hz ist.

3. Verfahren nach einem der vorhergehenden Ansprüche, bei dem die Charakterisierung eine Schätzung des systolischen Blutdrucks (PAS) umfasst, wobei das Verfahren, während der Inflationsphase, umfasst:

   - Detektion eines Stabilisierungszeitpunkts (ts), jenseits dessen sich die Niederfrequenzkomponente ($I_{DC}(t)$) stabilisiert und, nach dem Stabilisierungszeitpunkt, unter Bildung eines Plateaus verläuft;
   - Bestimmen des Drucks ($P(t_s)$), der zu dem Stabilisierungszeitpunkt ausgeübt wird.

4. Verfahren nach Anspruch 3, umfassend:

   - Berechnen einer Ableitung der Niederfrequenzkomponente ($I'_{DC}(t)$);
   - so dass der Stabilisierungszeitpunkt ($t_s$) dem Zeitpunkt entspricht, zu dem, während der Inflation, der absolute Wert der Ableitung ($|I'_{DC}(t)|$) kleiner als ein Stabilisierungsschwellenwert ($Th_s$) wird.

5. Verfahren nach einem der vorhergehenden Ansprüche, bei dem die Charakterisierung eine Schätzung des systolischen Blutdrucks (PAS) umfasst, wobei das Verfahren, während der Deflationsphase, umfasst:

   - Detektion eines Flankenzeitpunkts ($t_f$), zu dem die Niederfrequenzkomponente ($I_{DC}(t)$) eine ausgeprägte Sinkphase nach einer Stabilitätsphase beginnt;
   - Bestimmen des Drucks ($P(t_f)$), der zu dem Flankenzeitpunkt ausgeübt wird.

6. Verfahren nach Anspruch 5, umfassend:

   - Berechnen einer Ableitung der Niederfrequenzkomponente ($I'_{DC}(t)$);
   - so dass der Flankenzeitpunkt ($t_f$) dem Zeitpunkt entspricht, zu dem, während der Deflation, der absolute Wert der Ableitung ($|I'_{DC}(t)|$) größer als ein Flankenschwellenwert ($Th_f$) wird.

**7.** Verfahren nach einem der vorhergehenden Ansprüche, bei dem die Charakterisierung eine Schätzung des mittleren Blutdrucks (PAM) umfasst, wobei das Verfahren, während der Deflationsphase, umfasst:

- Detektion eines Zeitpunkts $^{TM}$, zu dem die Niederfrequenzkomponente ($I_{DC}(t)$) eine maximale Neigung erreicht;
- Bestimmen des Drucks ($P(t_m)$), der zu dem Zeitpunkt $^{TM}$ ausgeübt wird.

**8.** Verfahren nach Anspruch 5, umfassend:

- Berechnen einer Ableitung der Niederfrequenzkomponente ($I'_{DC}(t)$);
- so dass der Schätzzeitpunkt ($t_m$) des mittleren Blutdrucks einem Zeitpunkt entspricht, zu dem, während der Deflation, der absolute Wert der Ableitung ($|I'_{DC}(t)|$) maximal ist.

**9.** Verfahren nach einem der Ansprüche 3 bis 6 und nach Anspruch 7, umfassend eine Schätzung eines diastolischen Blutdrucks (PAD) ausgehend von dem systolischen Blutdruck (PAS) und dem mittleren Blutdruck (PAM).

**10.** Verfahren nach einem der vorhergehenden Ansprüche, bei dem die Charakterisierung eine Schätzung eines Zeitpunkts ($t_v$) umfasst, zu dem die Niederfrequenzkomponente ($I_{DC}(t)$), während der Deflation, einen minimalen Wert erreicht, wobei dieser Zeitpunkt einem Zeitpunkt des venösen Rückstroms entspricht.

**11.** Verfahren nach Anspruch 10, umfassend eine Bestimmung des Drucks ($P(t_v)$), der zu dem Zeitpunkt des venösen Rückstroms ausgeübt wird.

**12.** Verfahren nach einem der vorhergehenden Ansprüche, bei dem beim Schritt c) das Beleuchten der Gliedmaße in einem spektralen Beleuchtungsband in einem spektralen Band zwischen 500 nm und 1200 nm erfolgt.

**13.** Optische Vorrichtung (1) zur Charakterisierung eines Blutdrucks, wobei die Vorrichtung umfasst:

- ein Band (3), das dazu ausgestaltet ist, die optische Vorrichtung an einer Gliedmaße (2) eines Benutzers zu fixieren;
- eine Lichtquelle (10), die dazu ausgestaltet ist, ein Licht (12) zu emittieren, das sich zu der Gliedmaße des Benutzers hin ausbreitet, wenn die Vorrichtung getragen wird;
- einen Fotodetektor (20), der dazu eingerichtet ist, ein Licht (14) zu detektieren, das von der Lichtquelle emittiert wird und sich in der Gliedmaße ausgebreitet hat;

- eine Steuereinheit (30), die dazu programmiert ist, die Schritte d) bis e) nach einem der vorhergehenden Ansprüche ausgehend von dem von dem Fotodetektor detektierten Licht durchzuführen.

**Claims**

**1.** Method for achieving characterization of a blood pressure of a user, comprising the following steps:

- a) placing an optical device (1) on a limb (2) of the user, the optical device comprising:

• a light source (10), arranged to emit light (12) towards the limb;
• a photodetector (20), the photodetector being arranged to detect an intensity of light (14) emitted by the light source and having propagated through the limb;

- b) applying compression to the limb of the user, the compression being exerted between the heart of the individual and the optical device (1), such that a pressure ($P(t)$) is applied to the limb for a compression duration ($\Delta t$), the compression duration comprising:

• a phase of inflation, in which the applied pressure increases;
• a phase of deflation, in which the applied pressure decreases;

- c) during the compression duration ($\Delta t$), illuminating the limb with the light source and measuring an intensity detected by the photodetector at various times, so as to obtain a time-dependent function ($I(t)$) corresponding to a variation as a function of time in the intensity during the compression duration, the method being **characterized in that**:
- d) low-pass filtering is applied to the time-dependent function ($I(t)$), so as to extract a low-frequency component ($I_{DC}(t)$) from the time-dependent function ($I(t)$), the low-pass filtering being carried out with a cut-off frequency strictly lower than a heart rate of the individual;
- e) from a time determined from the low-frequency component ($I_{DC}(t)$), the blood pressure of the individual is characterized depending on the pressure applied at said time.

**2.** Method according to Claim 1, wherein the cut-off frequency is lower than 1 Hz or than 0.5 Hz.

**3.** Method according to either one of the preceding claims, wherein the characterization comprises estimating systolic blood pressure (PAS), the method comprising, during the inflation phase:

- detecting a stabilization time ($t_s$) beyond which the low-frequency component ($I_{DC}(t)$) stabilizes and varies, following the stabilization time, in such a way as to form a plateau;
- determining the pressure ($P(t_s)$) applied at the stabilization time.

4. Method according to Claim 3, comprising:

- computing a derivative ($I'_{DC}(t)$) of the low-frequency component;
- such that the stabilization time ($t_s$) corresponds to the time at which, during inflation, the absolute value ($|I'_{DC}(t)|$) of the derivative drops below a stabilization threshold ($Th_s$).

5. Method according to any one of the preceding claims, wherein the characterization comprises estimating systolic blood pressure (PAS), the method comprising, during the deflation phase:

- detecting an edge time ($t_f$) at which the low-frequency component ($I_{DC}(t)$) starts a phase of marked decrease following a phase of stability;
- determining the pressure ($P(t_f)$) applied at the edge time.

6. Method according to Claim 5, comprising:

- computing a derivative ($I'_{DV}(t)$) of the low-frequency component;
- such that the edge time ($t_f$) corresponds to the time at which, during deflation, the absolute value ($|I'_{DC}(t)|$) of the derivative rises above an edge threshold ($Th_f$).

7. Method according to any one of the preceding claims, wherein the characterization comprises estimating mean arterial pressure (PAM), the method comprising, during the deflation phase:

- detecting a time ($t_m$) at which the low-frequency component ($I_{DC}(t)$) reaches a maximum slope;
- determining the pressure ($P(t_m)$) applied at said time ($t_m$).

8. Method according to Claim 5, comprising:

- computing a derivative ($I'_{DC}(t)$) of the low-frequency component;
- such that the time ($t_m$) of estimation of the mean arterial pressure corresponds to a time at which, during deflation, the absolute value ($|I'_{DC}(t)|$) of the derivative is maximum.

9. Method according to any one of Claims 3 to 6 and according to Claim 7, comprising estimating a diastolic blood pressure (PAD) from the systolic blood pressure (PAS) and mean arterial pressure (PAM).

10. Method according to any one of the preceding claims, wherein the characterization comprises estimating a time ($t_v$) at which the low-frequency component ($I_{DC}(t)$) reaches a minimum value, during deflation, this time corresponding to a time of venous return.

11. Method according to Claim 10, comprising determining the pressure ($P(t_v)$) applied at the time of venous return.

12. Method according to any one of the preceding claims, wherein, in step c), the limb is illuminated in an illumination spectral band in a spectral band comprised between 500 nm and 1200 nm.

13. Optical device (1) for achieving characterization of a blood pressure, the device comprising:

- a fastening (3), configured to fasten the optical device to a limb (2) of a user;
- a light source (10), configured to emit light (12) that propagates towards the limb of the user when the device is being worn;
- a photodetector (20), arranged to detect light (14) emitted by the light source and having propagated through the limb of the user;
- a control unit (30), programmed to implement steps d) to e) according to any one of the preceding claims, on the basis of the light detected by the photodetector.

**Fig. 1A**

**Fig. 1B**

**Fig. 1C**

**Fig. 2A**

PPG oscillations

**Fig. 2B**

Fig. 3A

Fig. 3B

**Fig. 4**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20100160798 A

**Littérature non-brevet citée dans la description**

- **LUBIN M. et al.** Blood pressure measurement by coupling an external pressure and photo-plethysmographic signals. *EMBC*, July 2020